# EUROPÄISCHE PATENTSCHRIFT

(11) **EP 3 301 642 B1**
(45) Veröffentlichungstag und Bekanntmachung des Hinweises auf die Patenterteilung: **15.07.2020**
(21) Anmeldenummer: 17208544.1
(22) Anmeldetag: 19.12.2017
(51) Int. Cl.: G06T 7/00

(54) **AUTOMATISIERTE BILDPRÜFUNG IN DER RÖNTGENBILDGEBUNG**
AUTOMATED IMAGE VERIFICATION IN X-RAY IMAGING
VÉRIFICATION D'IMAGE AUTOMATISÉE DANS L'IMAGERIE PAR RAYONS X

(30) Priorität: 27.12.2016 DE 102016226230
(43) Veröffentlichungstag der Anmeldung: 04.04.2018
(73) Patentinhaber: Siemens Healthcare GmbH, 91052 Erlangen (DE)
(72) Erfinder: Jerebko, Anna, 91353 Hausen (DE); Jörger, Clemens, 91301 Forchheim (DE); Nolte, Francois, 91099 Poxdorf (DE)

(56) Entgegenhaltungen:
- WO-A2-2004/086941
- JP-A- H05 130 986
- US-A1- 2014 072 192
- US-A1- 2017 178 320

## Beschreibung

Die Erfindung betrifft ein Verfahren zum automatisierten Prüfen von Röntgenbilddaten. Weiterhin betrifft die Erfindung ein Bildgebungsverfahren. Überdies betrifft die Erfindung eine Bilddatenprüfeinrichtung. Zudem betrifft die Erfindung eine Röntgenbildgebungseinrichtung.

In der digitalen Radiographie können Röntgenbildaufnahmen Qualitätsmängel aufweisen. Solche Qualitätsmängel können durch eine zu geringe Röntgenstrahlendosis, eine zu hohe Röntgenstrahlendosis, eine Patientenbewegung oder eine falsche Positionierung eines Patienten verursacht werden. Als Patient soll im Folgenden sowohl ein zu untersuchender Mensch als auch ein zu untersuchendes Tier verstanden werden. In einem solchen Fall kann es notwendig sein, die Röntgenbestrahlung zu wiederholen und ein zusätzliches Bild aufzunehmen, um zu garantieren, dass das Bild alle klinisch relevanten Körperteile für eine Diagnose zeigt. Das zuerst aufgenommene Bild wird dann nicht weiter verwendet und aus dem System entfernt.

Allerdings sollte eine erneute Exposition des Patienten mit Röntgenstrahlung vermieden werden, da damit eine zusätzliche Belastung des Patienten verbunden ist. Außerdem wird durch eine zusätzliche Bildaufnahme zusätzlich Zeit pro Patient in der bildgebenden Einrichtung benötigt. Die Entscheidung, ob eine Bildaufnahme wiederholt werden muss, muss in sehr kurzer Zeit während der Untersuchung des Patienten erfolgen. Typischerweise müssen etwa 1 bis 5 % der Bilder wiederholt aufgenommen werden. Die größte Wahrscheinlichkeitsquote für eine Wiederholung findet sich bei freier Bestrahlung, zum Beispiel mit kabellosen Detektoren.

Bisher erfolgt eine Überprüfung der Bilder durch den Röntgenassistent, welcher während einer Röntgenbildaufnahme in einem Kontrollraum zur Überwachung des betreffenden Röntgenbildgebungssystems sitzt. Der Röntgenassistent muss unter Zeitdruck die Bilder prüfen und eine Entscheidung treffen, ob eine Bildaufnahme wiederholt werden muss. Eine solche Prüfung ist also mit zusätzlichem Zeitaufwand und Personalaufwand verbunden, wobei auch fehlerhafte Einstufungen von Bildern durch den Röntgenassistenten nie ganz auszuschließen sind.

In US 2014/ 0 072 192 A1 wird eine Qualitätsprüfung von Hand durch einen Prüfer durchgeführt, die ermittelten Qualitätsdaten werden einem der geprüften Bilder zugeordnet und dieses wird mit den anderen geprüften Bildern in einer Qualitätssicherungsdatenbank abgespeichert.

Mithin besteht ein Problem darin, ein einfacheres und zeitsparenderes Prüfverfahren von Röntgenbildern zu entwickeln, welches sich mit geringerem Personalaufwand realisieren lässt.

Diese Aufgabe wird durch ein Verfahren zum automatisierten Prüfen von Röntgenbilddaten gemäß Patentanspruch 1, ein Bildgebungsverfahren gemäß Patentanspruch 9, eine Bilddatenprüfeinrichtung gemäß Patentanspruch 11 und eine Röntgenbildgebungseinrichtung gemäß Patentanspruch 12 gelöst.

Bei dem erfindungsgemäßen Verfahren zum automatisierten Prüfen von Röntgenbilddaten von einem Untersuchungsbereich eines Patienten werden Röntgenbilddaten von dem Untersuchungsbereich empfangen. Die Röntgenbilddaten können zum Beispiel direkt während eines Bildgebungsprozesses von einer Rekonstruktionseinheit einer Röntgenbildgebungseinrichtung empfangen werden. Die Röntgenbilddaten können auch aus einem Bilddatenspeicher stammen, in dem die Röntgenbilddaten nach Abschluss eines Bildgebungsprozesses abgespeichert wurden. Nachfolgend werden die Röntgenbilddaten segmentiert und es werden anatomische Strukturen in den einzelnen Segmenten detektiert. Die Segmentierung erfolgt vorzugsweise automatisch basierend auf einem maschinenlernenden Algorithmus, der an einer Vielzahl von klinischen Bildern trainiert wurde, um die Umrandungen von Organen zu erkennen. Dabei werden die Bildsegmente ermittelt, welche für eine spätere Diagnose relevant sind. Um sicherzugehen, dass die richtigen Organe erkannt wurden, kann auch ein Vergleich der detektierten anatomischen Strukturen mit einem Organprogramm erfolgen. Ein solches Organprogramm weist einen Satz von Parametern auf, der einer Röntgenbildgebungseinrichtung genau vorgibt, wie geröntgt werden soll. Typischerweise ist dieser Satz spezifisch für jedes Organ bzw. Körperteil (z.B. Unterarm, Knie, Thorax) und wird durch ein zentrales Röntgenplanungssystem (RIS) angewählt. Anhand der Parameter eines Organprogramms lässt sich also feststellen, welche anatomische Struktur mit aufzunehmenden Bilddaten erfasst werden soll. Die segmentierten Röntgenbilddaten werden dann mit Referenzbilddaten verglichen und es wird ermittelt, welche der Referenzbilddaten den segmentierten Röntgenbilddaten am nächsten kommen. Dieser Vergleich wird automatisiert mit Hilfe eines Bildvergleichsalgorithmus durchgeführt.
Die dabei verwendeten Vergleichskriterien können unterschiedliche Bildparameter und/oder den Bildern zugeordnete Informationen umfassen.

Die Referenzbilddaten werden dazu aus einer Referenz-Datenbank ausgelesen und mit den relevanten Bildsegmenten verglichen. Die Referenzbilddaten der Referenz-Datenbank weisen jeweils eine Qualitätsinformation bezüglich der Bildqualität der Referenzbilddaten auf. D.h., den einzelnen Datensätzen der Referenzbilddaten ist jeweils eine Information zugeordnet, welche darüber Auskunft gibt, ob die Referenzbilddaten für eine spätere Diagnose verwendbar waren oder als nicht dafür geeignet eingestuft wurden. Die Referenzbilddaten stammen von früheren Röntgenbildaufnahmen und sind zusammen mit den zugeordneten Qualitätsinformationen in der Referenz-Datenbank gespeichert. Schließlich wird auf Basis der Qualitätsinformation der ermittelten Referenzbilddaten entschieden, ob die aktuell empfangenen Röntgenbilddaten für eine spätere Diagnose geeignet sind oder verworfen werden müssen. Dabei bedeutet letzteres, dass die Röntgenbilddaten nicht für eine spätere Diagnose genutzt werden, aber durchaus als Referenzbilddaten in der Referenz-Datenbank gespeichert werden, um als Vergleichsdaten für spätere Röntgenbildaufnahmen dienen zu können. Vorteilhaft werden Röntgenassistenten von der Prüfung der Bildqualität während der Bildaufnahme entlastet. Weiterhin wird aufgrund des automatisierten Vergleichs Zeit bei der Bildgebung eingespart. Überdies werden aufgrund des automatisierten Vorgehens Fehler bei der Qualitätsbeurteilung aufgrund von Zeitdruck, wie sie bei der herkömmlichen Bildprüfung durch einen Röntgenassistenten vorkommen, vermieden. Mithin werden unnötige Wiederholungen von Bildaufnahmen, welche für den Patienten eine zusätzliche Dosisbelastung bedeuten, vermieden.

Bei dem erfindungsgemäßen Bildgebungsverfahren werden zunächst Röntgenprojektionsdaten von einem Untersuchungsbereich eines Patienten mit Hilfe einer Röntgenbildgebungseinrichtung erfasst. D.h., es werden Röntgenprojektionsbilder mit Hilfe von Röntgenstrahlen von dem Untersuchungsbereich erzeugt. Auf Basis der erfassten Röntgenprojektionsdaten werden weiterhin Röntgenbilddatendaten erzeugt. Dieser Vorgang kann zum Beispiel einen Rekonstruktionsschritt und/oder einen oder mehrere Bildbearbeitungsvorgänge, beispielsweise einen Filterungsvorgang, zur Bildkorrektur umfassen. Schließlich werden die Röntgenbilddaten unter Anwendung des erfindungsgemäßen Verfahrens zum automatisierten Prüfen von Röntgenbilddaten von einem Untersuchungsbereich eines Patienten geprüft und für den Fall, dass ihre Qualität als ausreichend ermittelt wird, zur Bildanzeige freigegeben.

Die erfindungsgemäße Bilddatenprüfeinrichtung weist eine Bilddatenerfassungseinheit zum Empfangen von Röntgenbilddaten von einem Untersuchungsbereich eines Patienten auf. Eine solche Bilddatenerfassungseinheit kann zum Beispiel eine Eingangsschnittstelle zum Empfangen von Röntgenbilddaten aufweisen. Die erfindungsgemäße Bilddatenprüfeinrichtung weist eine Segmentiereinheit zum Segmentieren der Röntgenbilddaten und Detektieren von anatomischen Strukturen in den einzelnen Segmenten auf. Teil der erfindungsgemäßen Bilddatenprüfeinrichtung ist außerdem eine Vergleichseinheit zum Ermitteln von den Röntgenbilddaten am nächsten kommenden Referenzbilddaten auf Basis eines Vergleichs der empfangenen Röntgenbilddaten mit Referenzbilddaten aus einer Referenz-Datenbank. Die Referenzbilddaten der Referenz-Datenbank weisen jeweils eine Qualitätsinformation bezüglich der Bildqualität der Referenzbilddaten auf. Zudem umfasst die erfindungsgemäße Bilddatenprüfeinrichtung eine Prüfeinheit. Die Prüfeinheit ist dazu eingerichtet einen Entscheidungsprozess durchzuführen, bei dem entschieden wird, ob die empfangenen Röntgenbilddaten qualitativ ausreichend sind oder verworfen werden. Die Entscheidung erfolgt auf Basis der Qualitätsinformation der ermittelten Referenzbilddaten.

Die erfindungsgemäße Röntgenbildgebungseinrichtung weist eine Scaneinheit zum Erfassen von Röntgenbilddaten von einem Untersuchungsbereich eines Patienten, eine Steuerungseinrichtung zum Ansteuern der Scaneinheit und eine erfindungsgemäße Bilddatenprüfeinrichtung auf.

Die wesentlichen Komponenten der erfindungsgemäßen Bilddatenprüfeinrichtung können zum überwiegenden Teil in Form von Softwarekomponenten ausgebildet sein. Dies betrifft insbesondere die Segmentiereinheit, die Vergleichseinheit und die Prüfeinheit. Grundsätzlich können diese Komponenten aber auch zum Teil, insbesondere wenn es um besonders schnelle Berechnungen geht, in Form von softwareunterstützter Hardware, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden. Die Bilddatenprüfeinrichtung kann Teil der Röntgenbildgebungseinrichtung sein oder über eine Netzwerk-Schnittstelle (z.B. DICOM) mit einer solchen Röntgenbildgebungseinrichtung elektrisch verbunden sein. Bei letzterer Variante ist das Potential der Nachrüstbarkeit besonders große, da die Bilddatenprüfeinrichtung, wenn sie in ein Netzwerk integriert ist, zum Prüfen von Bilddaten von mehreren Röntgenbildgebungseinrichtungen, welche an das Netzwerk angeschlossen sind, genutzt werden kann.

Eine weitgehend softwaremäßige Realisierung hat den Vorteil, dass auch schon bisher verwendete Steuerungseinrichtungen von Röntgenbildgebungseinrichtungen auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer Röntgenbildgebungseinrichtung ladbar ist, mit Programmabschnitten, um alle Schritte des erfindungsgemäßen Verfahrens auszuführen, wenn das Computerprogramm in der Röntgenbildgebungseinrichtung ausgeführt wird. Wie bereits erwähnt, kann ein solches Computerprogramm auch anstatt direkt in einer Röntgenbildgebungseinrichtung gespeichert zu sein, in einem an ein Datenübertagungsnetzwerk angeschlossenen Rechner abgespeichert sein, welcher dazu dient, Bilddaten von verschiedenen, an das Datenübertragungsnetzwerk angeschlossenen Bildgebungseinrichtungen zu prüfen.

Ein solches Computerprogrammprodukt kann neben dem Computerprogramm gegebenenfalls zusätzliche Bestandteile wie z.B. eine Dokumentation und/oder zusätzliche Komponenten auch Hardware-Komponenten, wie z.B. Hardware-Schlüssel (Dongles etc.) zur Nutzung der Software, umfassen

Zum Transport zu einer Speichereinrichtung einer solchen Röntgenbildgebungseinrichtung und/oder zur Speicherung an der Röntgenbildgebungseinrichtung oder gegebenenfalls in einem Rechner eines Datenübertragungsnetzwerks kann ein computerlesbares Medium, beispielsweise ein Memorystick, eine Festplatte oder ein sonstiger transportabler oder fest eingebauter Datenträger dienen, auf welchem die von einer Rechnereinheit einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Die abhängigen Ansprüche sowie die nachfolgende Beschreibung enthalten jeweils besonders vorteilhafte Ausgestaltungen und Weiterbildungen der Erfindung. Dabei können insbesondere die Ansprüche einer Anspruchskategorie auch analog zu den abhängigen Ansprüchen einer anderen Anspruchskategorie oder deren Beschreibungsteilen weitergebildet sein. Zudem können im Rahmen der Erfindung die verschiedenen Merkmale unterschiedlicher Ausführungsbeispiele und Ansprüche auch zu neuen Ausführungsbeispielen kombiniert werden.

In einer Ausgestaltung des erfindungsgemäßen Verfahrens zum automatisierten Prüfen von Röntgenbilddaten von einem Untersuchungsbereich eines Patienten erfolgt der Vergleich der segmentierten Röntgenbilddaten mit Referenzbilddaten anhand einer detektierten, interessierenden anatomischen Struktur in den segmentierten Röntgenbilddaten. D.h., es wird eine anatomische Struktur in den aktuell aufgenommenen Röntgenbilddaten mit einer entsprechenden anatomischen Struktur in den Referenzbilddaten verglichen.

Ein solcher Vergleich auf Basis einer detektierten, interessierenden anatomischen Struktur in den segmentierten Röntgenbilddaten mit den Referenzbilddaten kann zum Beispiel anhand mindestens eines der folgenden Kriterien erfolgen:
- der Form der Struktur,
- der Größe der Struktur,
- der Absorptionsverteilung in der Struktur,
- der Existenz von Implantaten in der Struktur und dem Typ der Implantate,
- dem Auftreten von Todesfällen, Frakturen,
- dem Bildkontext,
- der Verteilung der Graustufen in dem Bild,
- Aufnahmeparametern.

Es können auch Kombinationen der genannten Kriterien verwendet werden, um geeignete Referenzbilddaten zu finden. Auf diese Weise kann ein Referenzbilddatensatz aus der Referenz-Datenbank herausgesucht werden, der aktuellen Röntgenbildaufnahme am nächsten kommt. Mithin ist die Zuverlässigkeit des Prüfungsergebnisses entsprechend erhöht.

Bevorzugt werden bei dem erfindungsgemäßen Verfahren zum automatisierten Prüfen von Röntgenbilddaten von einem Untersuchungsbereich eines Patienten bei dem Ermitteln von nächstkommenden Referenzbilddaten ein oder mehrere Referenzbilddatensätze ermittelt und es werden die empfangenen Röntgenbilddaten beibehalten, wenn mindestens einer der Referenzbilddatensätze nicht verworfen wurde, d.h. die Qualität mindestens eines der Referenzbilddatensätze als ausreichend deklariert wurde. Es kann vorkommen, dass eine Mehrzahl von Referenzbilddatensätzen dem aktuellen Bilddatensatz ähnlich nahe kommen. Dann genügt es, wenn einer der Referenzbilddatensätze als qualitativ ausreichend eingestuft wurde. Es kann auch der Fall sein, dass im Falle von Krankheitsbildern (z.B. Knie mit Arthrose versus Knie ohne Arthrose) mehrere Bilddatensätze unterschiedliche Ergebnisse liefern, obwohl sie röntgentechnisch ähnlich sind. In diesem Fall wird dasjenige Referenzbild als Vergleichsbild ausgewählt, welches demselben Krankheitsbild wie die zu prüfenden Bilddaten zugeordnet ist. Auf diese Weise wird die Vergleichbarkeit der ausgewählten Referenzbilddaten erhöht und die Zuverlässigkeit eines Prüfergebnisses verbessert.

Besonders bevorzugt erfolgt der Vergleich auf Basis einer detektierten, interessierenden anatomischen Struktur in den segmentierten Röntgenbilddaten mit den Referenzbilddaten auf Basis von klinischen Patientendaten. Klinische Patientendaten können zum Beispiel Voraufnahmen, demographische Daten, weitere Diagnosen oder Daten betreffend die Anamnese umfassen. Diese Daten können zum Beispiel kombiniert als Zusatz zu den weiter oben beschriebenen bildtechnischen Daten für den Vergleich genutzt werden, was zu einer besseren Vergleichbarkeit der ermittelten Referenzbilddaten beiträgt.

In einer besonders vorteilhaft anzuwendenden Variante des erfindungsgemäßen Verfahrens zum automatisierten Prüfen von Röntgenbilddaten von einem Untersuchungsbereich eines Patienten werden nächstkommende Referenzbilddaten mit Hilfe eines automatisierten Vergleichsverfahrens ermittelt. Wie bereits erwähnt, können für die Auswahl geeigneter Referenzbilddaten Bildstrukturdaten und auch zusätzliche klinische Patientendaten genutzt werden. Bei einer automatisierten Durchführung des Vergleichs kann Zeit und Personalaufwand eingespart werden und die Auswahl der Referenzbilddaten unabhängig von individuellen Präferenzen des Personals vorgenommen werden, wodurch das Prüfverfahren objektiver gestaltet werden kann.

In einer besonders vorteilhaften Ausgestaltung des erfindungsgemäßen Verfahrens zum automatisierten Prüfen von Röntgenbilddaten von einem Untersuchungsbereich eines Patienten wird die Entscheidung, ob die empfangenen Röntgenbilddaten verworfen werden, in Abhängigkeit davon getroffen, ob eine Systemeinstellung, d.h. eine Einstellung von Parametern eines Bildaufnahmevorgangs, geändert wurde. Werden zum Beispiel bei einer Bildaufnahmesequenz für eine Bildaufnahme andere Systemparameter, wie zum Beispiel ein geänderter Aufnahmewinkel, genutzt, so kann eine Änderung dieser Größe innerhalb einer Bildaufnahmesequenz darauf hindeuten, dass dieser Parameter während der Aufnahme unabsichtlich verstellt wurde. Jedenfalls passt das Bild eventuell nicht zu den anderen Bildern der Aufnahmesequenz und kann aussortiert werden.

Im Rahmen einer bevorzugten Variante des erfindungsgemäßen Verfahrens zum automatisierten Prüfen von Röntgenbilddaten von einem Untersuchungsbereich eines Patienten wird für den Fall, dass entschieden wurde, dass die empfangenen Röntgenbilddaten verworfen werden, eine der folgenden Informationen an den Benutzer übermittelt:
- Informationen bezüglich der Gründe für die Entscheidung,
- Benutzeranweisungen, um die Positionierung des Patienten und/oder von Teilen einer zur Aufnahme der Röntgenbilddaten eingesetzten Röntgenbildgebungseinrichtung zu ändern,
- Statistikdaten zu verworfenen Bilddaten als Warnung für das zukünftige Aufnehmen von entsprechenden Röntgenbilddaten.

Zusätzlich zu der Information bezüglich der Qualitätseigenschaften der aktuell aufgenommenen Röntgenbilddaten erhält der Benutzer also noch Informationen, welche er dazu nutzen kann, bei einer wiederholten Bildaufnahme ein besseres Ergebnis zu erzielen. Mithin wird der Bildaufnahmeprozess weiter beschleunigt, die Dosisbelastung des Patienten reduziert und der Aufnahmekomfort aufgrund der kürzeren Zeitdauer erhöht.

In einer speziellen Variante des erfindungsgemäßen Bildgebungsverfahrens wird für den Fall, dass bei dem Prüfschritt entschieden wurde, dass die empfangenen Röntgenbilddaten verworfen werden, eine der folgenden Anweisungen an eine Steuereinrichtung der Röntgenbildgebungseinrichtung übermittelt:
- Anweisungen zur automatisierten Bildbearbeitung des verworfenen Bilds,
- Anweisungen zur automatisierten Korrektur der Positionierung des Patienten und/oder von Teilen der Röntgenbildgebungseinrichtung.

Vorteilhaft werden also automatisiert Korrekturen zur Verbesserung der Bildqualität vorgenommen, so dass der Benutzer nicht eingreifen muss. Mithin ist das Bildaufnahmeergebnis weniger abhängig von der Kompetenz des Bedienpersonals, so dass eine ausreichende Bildqualität auch bei schlechter ausgebildetem Personal garantiert wird.

Die Erfindung wird im Folgenden unter Hinweis auf die beigefügten Figuren anhand von Ausführungsbeispielen noch einmal näher erläutert. Es zeigen:
FIG 1 ein Flussdiagramm, welches ein Verfahren zum automatisierten Prüfen von Röntgenbilddaten von einem Untersuchungsbereich eines Patienten veranschaulicht,
FIG 2 ein Blockdiagramm, mit dem eine Bilddatenprüfeinrichtung gemäß einem Ausführungsbeispiel der Erfindung veranschaulicht wird,
FIG 3 ein Blockdiagramm, mit dem eine Röntgenbildgebungseinrichtung gemäß einem Ausführungsbeispiel der Erfindung dargestellt wird.

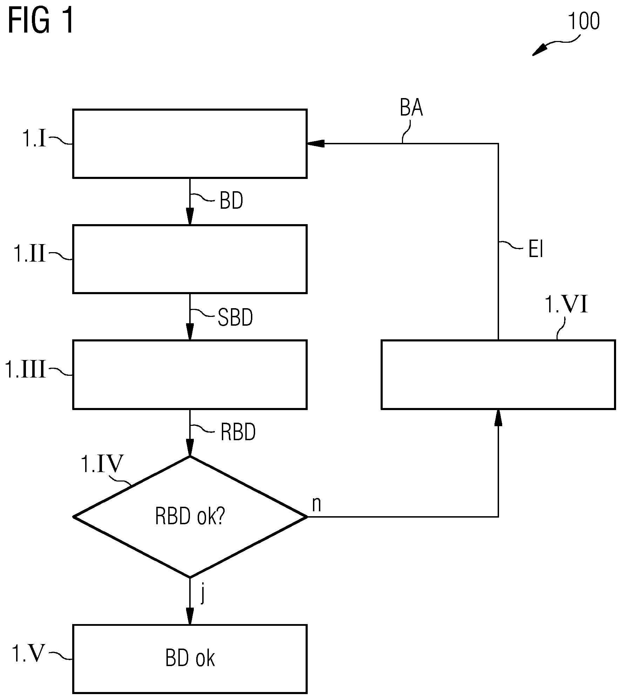

In FIG 1 ist ein Flussdiagramm 100 gezeigt, mit dem ein Verfahren zum automatisierten Prüfen von Röntgenbilddaten BD von einem Untersuchungsbereich eines Patienten gemäß einem Ausführungsbeispiel der Erfindung veranschaulicht wird. Bei dem Schritt 1.I werden Röntgenbilddaten BD von einem Untersuchungsbereich eines Patienten erfasst. Die Röntgenbilddaten BD können zum Beispiel direkt während eines Bildgebungsprozesses von einer Auswertungseinheit einer Röntgenbildgebungseinrichtung empfangen werden. Die Röntgenbilddaten BD können auch aus einem Bilddatenspeicher stammen, in dem die Röntgenbilddaten BD nach Abschluss eines Bildgebungsprozesses abgespeichert wurden. Bei dem Schritt 1.II wird eine Segmentierung der empfangenen Röntgenbilddaten BD durchgeführt. Dabei wird ein den Röntgenbilddaten BD entsprechendes Röntgenbild in Segmente aufgeteilt, die jeweils einzelnen Organen oder Organgruppen zugeordnet sind, und es werden segmentierte Bilddaten SBD erzeugt. Die Segmentierung erfolgt automatisch basierend auf einem maschinenlernenden Algorithmus, der an einer Vielzahl von klinischen Bildern trainiert wurde, um die Umrandungen von Organen zu erkennen. Im Rahmen der Segmentierung werden bei dem Schritt 1.II auch die den einzelnen Segmenten zugeordneten anatomischen Strukturen ermittelt bzw. ergeben sich aus dem maschinell durchgeführten Segmentierungsprozess, bei dem einzelnen anatomische Strukturen erkannt und segmentiert werden.

Anschließend wird bei dem Schritt 1.III auf Basis der detektierten Strukturen ein Vergleich der Röntgenbilddaten BD bzw. der bei dem Schritt 1.II bereits segmentierten Bilddaten SBD mit Referenzbilddatensätzen RBD aus einer Datenbank vorgenommen. Die Referenzbilddatensätze RBD umfassen Röntgenbilddaten von älteren Bildaufnahmen. Zusätzlich sind den einzelnen Referenzbilddatensätzen RBD jeweils Informationen bezüglich der Frage zugeordnet, ob die Bilddaten aufgrund von Qualitätsmängeln verworfen wurden oder eine ausreichende Qualität aufweisen, so dass sie als Grundlage für eine spätere Diagnose genutzt werden konnten. Bei dem Schritt 1.III wird derjenige Referenzbilddatensatz RBD aus der Datenbank ermittelt, welcher den ermittelten Strukturen am nächsten kommt. Bei dem Schritt 1.IV werden nun die dem ermittelten Referenzbilddatensatz RBD zugeordneten Zusatzinformationen ausgewertet. Geben diese darüber Auskunft, dass der Referenzbilddatensatz RBD in Ordnung ist, d.h. dass der Referenzbilddatensatz RBD nicht verworfen wurde, sondern als für eine spätere Diagnose geeignet eingestuft wurde, so wird dieses Ergebnis auf den zu prüfenden Bilddatensatz BD übertragen. Wird also der zu prüfende Bilddatensatz BD als geeignet eingestuft, was in FIG 1 mit "j" gekennzeichnet ist, so wird zu dem Schritt 1.V übergegangen und dieser Bilddatensatz BD für eine spätere Diagnose gespeichert. Für den Fall, dass bei dem Schritt 1.IV ermittelt wurde, dass der bei dem Vergleich verwendete Referenzbilddatensatz RBD verworfen wurde, was in FIG 1 mit "n" gekennzeichnet wurde, so wird zu dem Schritt 1.VI übergegangen. Bei dem Schritt 1.VI wird der zu prüfende Bilddatensatz BD verworfen und es werden Anweisungen BA zur Korrektur von Akquisitionsparametern erteilt, um bei einer Wiederholung der Bildaufnahme verbesserte Bilddaten zu erhalten. Beispielsweise umfassen die Akquisitionsparameter eine geänderte Patientenposition oder eine geänderte Position einzelner Teile einer zur Bildaufnahme verwendeten bildgebenden Einrichtung. Anschließend wird zu dem Schritt 1.I zurückgekehrt und erneut eine Bildaufnahme von dem Untersuchungsbereich vorgenommen und nachfolgend werden die weiteren, bereits beschriebenen Schritte des Verfahrens wiederholt.

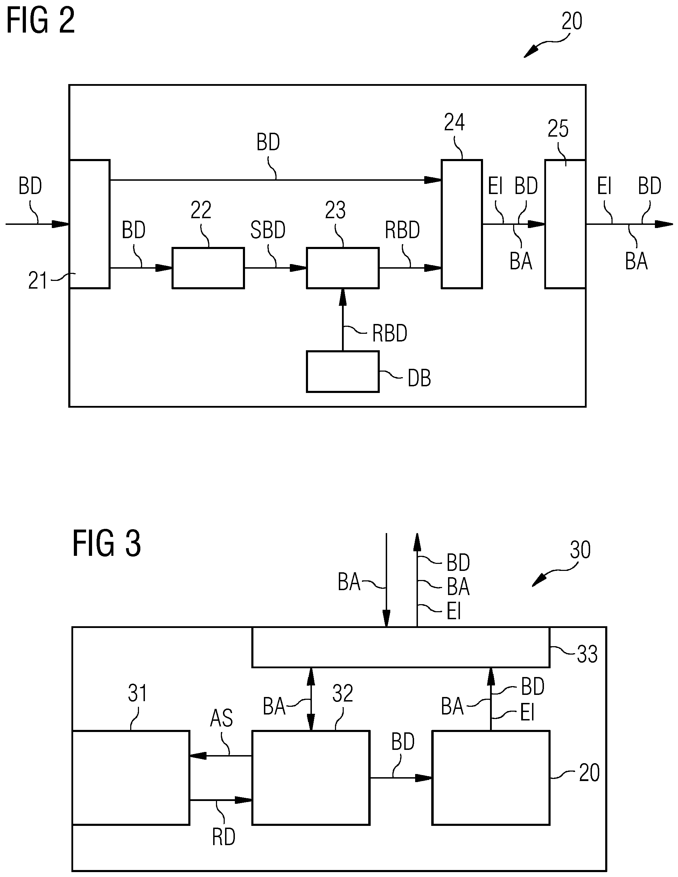

In FIG 2 ist eine Bilddatenprüfeinrichtung 20 gemäß einem Ausführungsbeispiel der Erfindung gezeigt. Die Bilddatenprüfeinrichtung 20 umfasst eine Eingangsschnittstelle 21, welche Bildddaten BD empfängt und an eine Segmentiereinheit 22 sowie eine Prüfeinheit 24 übermittelt. Die Segmentiereinheit 22 erzeugt segmentierte Bilddaten SBD, wobei einzelne Bildsegmente einzelnen Organen oder Organgruppen zugeordnet werden. Bei der Segmentierung wird ein Bildbereich der Bilddaten BD markiert, welcher einem für eine spätere Diagnose relevanten Bereich entspricht. Dieser Bereich kann zum Beispiel ein bestimmtes Organ oder Körperteil oder eine bestimmte Organgruppe umfassen, welche bei einer späteren Diagnose eingehend untersucht werden sollen. Dieser segmentierte Bereich SBD wird dann von einer Vergleichseinheit 23 mit einer Mehrzahl von Referenzbilddatensätzen RBD verglichen, welche in einer Datenbank DB abgespeichert sind. Das Referenzbild RBD, welches einen Bildabschnitt umfasst, welcher dem segmentierten Bereich SBD am nächsten kommt, wird ausgesucht und an eine Prüfeinheit 24 übermittelt.

Die Prüfeinheit 24 liest dem ermittelten Referenzbild RBD zugeordnete Qualitätsinformationen aus und entscheidet auf dieser Basis, ob die aktuell erfassten Bilddaten BD zu verwerfen sind oder nicht. Eine entsprechende Ergebnisinformation EI wird über eine Ausgangsschnittstelle 25 an einen Benutzer und/oder eine Steuerungseinrichtung einer bildgebenden Einrichtung (siehe FIG 3) ausgegeben. Für den Fall eines positiven Ergebnisses, d.h. für den Fall, dass das zu prüfende Bild BD vorbestimmte Bildqualitätsanforderungen erfüllt, d.h. von der Prüfeinheit 24 nicht verworfen wurde, werden die aktuell erfassten Bilddaten BD ebenfalls über die Ausgangsschnittstelle 25 ausgegeben. Für den Fall, dass das Prüfergebnis negativ ist, d.h. für den Fall, dass das zu prüfende Bild BD vorbestimmte Bildqualitätsanforderungen nicht erfüllt und mithin von der Prüfeinheit 24 verworfen wurde, werden statt der aktuell erfassten Bilddaten BD Anweisungen BA über die Ausgangsschnittstelle 25 ausgegeben, mit deren Hilfe eine Korrektur der Akquisitionsparameter der Bilderfassung vorgenommen werden kann und damit die Bildaufnahme wiederholt werden kann.

In FIG 3 ist eine Röntgenbildgebungseinrichtung 30 gemäß einem Ausführungsbeispiel der Erfindung gezeigt. Eine solche Röntgenbildgebungseinrichtung kann zum Beispiel als Computertomographieeinrichtung ausgebildet sein. Die Röntgenbildgebungseinrichtung 30 umfasst eine Scaneinheit 31 zum Erfassen von Röntgenbilddaten BD von einem Untersuchungsbereich eines Patienten. Weiterhin ist Teil der Röntgenbildgebungseinrichtung 30 auch eine Steuerungseinrichtung 32, mit der Ansteuerbefehle AS an die Scaneinheit 31 übergeben werden und Röntgenprojektionsmessdaten RD empfangen und zu Bilddaten BD verarbeitet werden. Die Bilddaten BD werden an eine Bilddatenprüfeinrichtung 20 übermittelt, welche die Bilddaten BD auf die im Zusammenhang mit FIG 1 und FIG 2 beschriebene Weise prüft und entsprechende Ergebnisinformationen EI und gegebenenfalls Bilddaten BD oder Korrekturanweisungen BA über eine Benutzerschnittstelle 33 ausgibt. Die Korrekturanweisungen BA können dazu genutzt werden die Scaneinheit 31 mit geänderten Akquisitionsparametern AS anzusteuern und auf diese Weise eine verbesserte Bildaufnahme zu erzielen.

Es wird abschließend noch einmal darauf hingewiesen, dass es sich bei den vorbeschriebenen Verfahren und Vorrichtungen lediglich um bevorzugte Ausführungsbeispiele der Erfindung handelt und dass die Erfindung vom Fachmann variiert werden kann, ohne den Bereich der Erfindung zu verlassen, soweit er durch die Ansprüche vorgegeben ist. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können.

### Bezugszeichenliste

- AS: Ansteuerbefehle
- BA: Benutzeranweisungen
- BD: Röntgenbilddaten
- DB: Datenbank
- EI: Ergebnisinformation
- RBD: Referenzbilddatensätzen
- RD: Röntgenprojektionsdaten
- SBD: segmentierte Bilddaten

- 20: Bilddatenprüfeinrichtung
- 21: Eingangsschnittstelle
- 22: Segmentiereinheit
- 23: Vergleichseinheit
- 24: Prüfeinheit
- 25: Ausgangsschnittstelle
- 30: Röntgenbildgebungseinrichtung
- 31: Scaneinheit
- 32: Steuerungseinrichtung
- 33: Benutzerschnittstelle

## Patentansprüche

1. Verfahren zum automatisierten Prüfen von Röntgenbilddaten (BD) von einem Untersuchungsbereich eines Patienten, aufweisend die Schritte:
- Empfangen von Röntgenbilddaten (BD) von dem Untersuchungsbereich,
- Segmentieren der Röntgenbilddaten (BD) und Detektieren von anatomischen Strukturen in den einzelnen Segmenten,
- Ermitteln von den segmentierten Röntgenbilddaten (SBD) am nächsten kommenden Referenzbilddaten (RBD) auf Basis eines Vergleichs der segmentierten Röntgenbilddaten (SBD) mit Referenzbilddaten (RBD) aus einer Referenz-Datenbank (DB), wobei die Referenzbilddaten (RBD) der Referenz-Datenbank (DB) jeweils eine Qualitätsinformation bezüglich der Bildqualität der Referenzbilddaten (RBD) hinsichtlich der Fragestellung, ob die Referenzbilddaten (RBD) für eine spätere Diagnose verwendbar waren oder als nicht dafür geeignet eingestuft wurden, aufweisen,
- Entscheiden, ob die empfangenen Röntgenbilddaten (BD) beibehalten oder verworfen werden, auf Basis der Qualitätsinformation der ermittelten Referenzbilddaten (RBD).

2. Verfahren nach Anspruch 1, wobei der Vergleich der segmentierten Röntgenbilddaten (SBD) mit Referenzbilddaten (RBD) anhand einer detektierten, interessierenden anatomischen Struktur in den segmentierten Röntgenbilddaten (SBD) erfolgt.

3. Verfahren nach Anspruch 2, wobei der Vergleich auf Basis einer detektierten, interessierenden anatomischen Struktur in den segmentierten Röntgenbilddaten (SBD) mit den Referenzbilddaten (RBD) anhand mindestens eines der folgenden Kriterien erfolgt:
- Form der Struktur,
- Größe der Struktur,
- Absorptionsverteilung in der Struktur,
- Existenz von Implantaten in der Struktur und Typ der Implantate,
- Auftreten von Todesfällen, Frakturen,
- Bildkontext,
- Verteilung der Graustufen in dem Bild,
- Aufnahmeparameter.

4. Verfahren nach einem der Ansprüche 1 bis 3, wobei der Vergleich auf Basis einer detektierten, interessierenden anatomischen Struktur in den segmentierten Röntgenbilddaten (SBD) mit den Referenzbilddaten (RBD) anhand von klinischen Patientendaten erfolgt.

5. Verfahren nach einem der vorstehenden Ansprüche, wobei bei dem Ermitteln von nächstkommenden Referenzbilddaten (RBD) ein oder mehrere Referenzbilddatensätze (RBD) ermittelt werden und die empfangenen Röntgenbilddaten (BD) nicht verworfen werden, wenn mindestens einer der Referenzbilddatensätze (RBD) nicht verworfen wurde.

6. Verfahren nach einem der vorstehenden Ansprüche, wobei das Ermitteln von nächstkommenden Referenzbilddaten (RBD) mit Hilfe eines automatisierten Vergleichsverfahrens erfolgt.

7. Verfahren nach einem der vorstehenden Ansprüche, wobei die Entscheidung, ob die empfangenen Röntgenbilddaten (BD) verworfen werden, in Abhängigkeit davon getroffen wird, ob eine Einstellung von Parametern eines Bildaufnahmevorgangs geändert wurde.

8. Verfahren nach einem der vorstehenden Ansprüche, wobei für den Fall, dass entschieden wurde, dass die empfangenen Röntgenbilddaten (BD) verworfen werden, eine der folgenden Informationen an den Benutzer übermittelt wird:
- Informationen bezüglich der Gründe für die Entscheidung,
- Benutzeranweisungen (BA), um die Positionierung des Patienten und/oder von Teilen einer zur Aufnahme der Bilddaten verwendeten Röntgenbildgebungseinrichtung (30) zu ändern,
- Statistikdaten zu verworfenen Bilddaten (BD) als Warnung für das zukünftige Aufnehmen von entsprechenden Röntgenbilddaten (BD).

9. Bildgebungsverfahren, aufweisend die Schritte:
- Erfassen von Röntgenprojektionsdaten (RD) von einem Untersuchungsbereich eines Patienten mit Hilfe einer Röntgenbildgebungseinrichtung (30),
- Erzeugen von Röntgenbilddaten (BD) auf Basis der erfassten Röntgenprojektionsdaten (RD),
- Prüfen der Röntgenbilddaten (BD) unter Anwendung eines Verfahrens nach einem der Ansprüche 1 bis 8,
- Freigeben der Röntgenbilddaten (BD) zur Bildanzeige, für den Fall, dass die Qualität der Röntgenbilddaten (BD) als ausreichend ermittelt wurde.

10. Verfahren nach Anspruch 9, wobei für den Fall, dass bei dem Prüfschritt entschieden wurde, dass die empfangenen Röntgenbilddaten (BD) verworfen werden, eine der folgenden Anweisungen (AS) an eine Steuerungseinrichtung (32) der Röntgenbildgebungseinrichtung (30) übermittelt werden:
- Anweisungen zur automatisierten Bildbearbeitung des verworfenen Bilds,
- Anweisungen (AS) zur automatisierten Korrektur der Positionierung des Patienten und/oder von Teilen der Röntgenbildgebungseinrichtung (30).

11. Bilddatenprüfeinrichtung (20), aufweisend:
- eine Bilddatenerfassungseinheit (21) zum Empfangen von Röntgenbilddaten (BD) von einem Untersuchungsbereich eines Patienten,
- eine Segmentiereinheit (22) zum Segmentieren der Röntgenbilddaten (BD) und Detektieren von anatomischen Strukturen in den einzelnen Segmenten,
- eine Vergleichseinheit (23) zum Ermitteln von den Röntgenbilddaten (BD) am nächsten kommenden Referenzbilddaten (RBD) auf Basis eines Vergleichs der segmentierten Röntgenbilddaten (SBD) mit Referenzbilddaten (RBD) aus einer Referenz-Datenbank (DB), wobei die Referenzbilddaten (RBD) der Referenz-Datenbank (DB) jeweils eine Qualitätsinformation bezüglich der Bildqualität der Referenzbilddaten (RBD) hinsichtlich der Fragestellung, ob die Referenzbilddaten (RBD) für eine spätere Diagnose verwendbar waren oder als nicht dafür geeignet eingestuft wurden, aufweisen,
- eine Prüfeinheit (24) zum Entscheiden, ob die empfangenen Röntgenbilddaten (BD) verworfen werden, auf Basis der Qualitätsinformation der ermittelten Referenzbilddaten (RBD).

12. Röntgenbildgebungseinrichtung (30), aufweisend:
- eine Scaneinheit (31) zum Erfassen von Röntgenbilddaten (BD) von einem Untersuchungsbereich eines Patienten,
- eine Steuerungseinrichtung (32) zum Ansteuern der Scaneinheit (31),
- eine Bilddatenprüfeinrichtung (20) nach Anspruch 11.

13. Computerprogrammprodukt mit einem Computerprogramm, welches direkt in eine Speichereinheit einer Röntgenbildgebungseinrichtung (30) ladbar ist, mit Programmabschnitten, um alle Schritte des Verfahrens nach einem der Ansprüche 1 bis 10 auszuführen, wenn das Computerprogramm in der Röntgenbildgebungseinrichtung (30) ausgeführt wird.

14. Computerlesbares Medium, auf welchem von einer Recheneinheit ausführbare Programmabschnitte gespeichert sind, um alle Schritte des Verfahrens nach einem der Ansprüche 1 bis 10 auszuführen, wenn die Programmabschnitte von Recheneinheit ausgeführt werden.

## Claims

1. Method for automated evaluation of x-ray image data (BD) from an examination region of a patient, having the following steps:
- Receiving of x-ray image data (BD) from the examination region,
- Segmentation of the x-ray image data (BD) and detection of anatomical structures in the individual segments,
- Establishing of reference image data (RBD) coming closest to the segmented x-ray image data (SBD) on the basis of a comparison of the segmented x-ray image data (SBD) with reference image data (RBD) from a reference database (DB), wherein the reference image data (RBD) of the reference database (DB) has quality information relating to the image quality of the reference image data (RBD) with regard to the question of whether the reference image data (RBD) was able to be used for a later diagnosis or has been classified as not suitable for this in each case,
- Deciding whether the received x-ray image data (BD) will be retained or rejected, based on the quality information of the established reference image data (RBD).

2. Method according to claim 1, wherein the comparison of the segmented x-ray image data (SBD) with reference image data (RBD) is made on the basis of a detected anatomical structure of interest in the segmented x-ray image data (SBD).

3. Method according to claim 2, wherein the comparison is made on the basis of a detected, anatomical structure of interest in the segmented x-ray image data (SBD) with the reference image data (RBD) on the basis of at least one of the following criteria:
- Form of the structure,
- Size of the structure,
- Absorption distribution in the structure,
- Existence of implants in the structure and type of the implants,
- Occurrence of fatalities, fractures,
- Image context,
- Distribution of the grey levels in the image,
- Recording parameters.

4. Method according to one of claims 1 to 3, wherein the comparison is made on the basis of a detected anatomical structure of interest in the segmented x-ray image data (SBD) with the reference image data (RBD) on the basis of clinical patient data.

5. Method according to one of the preceding claims, wherein, when establishing closest reference image data (RBD), one or more reference image datasets (RBD) is established and the received x-ray image data (BD) will not be rejected, when at least one of the reference image datasets (RBD) has not been rejected.

6. Method according to one of the preceding claims, wherein the closest reference image data (RBD) is established with the aid of an automated comparison method.

7. Method according to one of the preceding claims, wherein the decision as to whether the received x-ray image data (BD) will be rejected is made on the basis of whether a system setting has been changed.

8. Method according to one of the preceding claims, wherein, in the event of it having been decided that the received x-ray image data (BD) will be rejected, one of the following items of information will be transferred to the user:
- Information relating to the reasons for the decision,
- User instructions (BA) for changing the positioning of the patient and/or of parts of an x-ray imaging device (30) used for recording the image data,
- Statistics data about rejected image data (BD) as a warning about the future recording of corresponding x-ray image data (BD) .

9. Imaging method, having the steps:
- Acquisition of the x-ray projection data (RD) from an examination region of a patient with the aid of an x-ray imaging device (30),
- Creation of x-ray image data (BD) on the basis of the acquired x-ray projection data (RD),
- Evaluation the x-ray image data (BD) using a method according to one of claims 1 to 8,
- Release of the x-ray image data (BD) for image display in the event of the quality of the x-ray image data (BD) having been established as adequate.

10. Method according to claim 9, wherein, in the event of it having been decided in the evaluation step that the received x-ray image data (BD) will be rejected, one of the following instructions (AS) will be transferred to a control device (32) of the x-ray imaging device (30):
- Instructions for automated image processing of the rejected image,
- Instructions (AS) for automated correction of the positioning of the patient and/or of parts of the x-ray imaging device (30).

11. Image data evaluation device (20), having:
- An image data acquisition unit (21) for receiving x-ray image data (BD) from an examination region of a patient,
- A segmentation unit (22) for segmentation of the x-ray image data (BD) and detection of anatomical structures in the individual segments,
- A comparison unit (23) for establishing reference image data (RBD) coming closest to the x-ray image data (BD) on the basis of a comparison of the segmented x-ray image data (SBD) with reference image data (RBD) from a reference database (DB), wherein the reference image data (RBD) of the reference database (DB) has quality information relating to the image quality of the reference image data (RBD) in each case with regard to the question of whether the reference image data (RBD) was able to be used for a later diagnosis or has been classified as not suitable for this,
- An evaluation unit (24) for deciding whether the received x-ray image data (BD) will be rejected, on the basis of the quality information of the established reference image data (RBD).

12. X-ray imaging device (30), having:
- A scan unit (31) for acquisition of x-ray image data (BD) from an examination region of a patient,
- A control device (32) for controlling the scan unit (31),
- An image data evaluation device (20) according to claim 11.

13. Computer program product with a computer program, which is able to be loaded directly into a memory unit of an x-ray imaging device (30), with program sections to carry out all steps of the method according to one of claims 1 to 10, when the computer program is executed in the x-ray imaging device (30) .

14. Computer-readable medium, on which program sections able to be executed by a processing unit are stored, to carry out all steps of the method according to one of claims 1 to 10, when the program sections are executed by the processing unit.

## Revendications

1. Procédé de contrôle automatisé de données (BD) d'image aux rayons X d'une région à examiner d'un patient, comportant les stades :
- réception de données (BD) d'image aux rayons X de la région à examiner,
- segmentation des données (BD) d'image aux rayons X et détection de structures anatomiques dans les divers segments,
- détermination des données (SBD) d'image aux rayons X segmentées à des données (RBD) d'image de référence venant immédiatement ensuite sur la base d'une comparaison des données (SBD) d'image aux rayons X segmentées à des données (RBD) d'image de référence provenant d'une base (DB) de données de référence, les données (RBD) d'image de référence de la base (DB) de données de référence ayant chacune une information de qualité concernant la qualité d'image des données (RBD) d'image de référence sur le point de savoir si les données (RBD) d'image de référence ont été classées comme pouvant être utilisées pour un diagnostic ultérieur ou comme n'y convenant pas,
- décision si les données (BD) d'image aux rayons X reçues sont conservées ou rejetées sur la base de l'information de qualité des données (RBD) d'image de référence qui ont été déterminées.

2. Procédé suivant la revendication 1, dans lequel la comparaison des données (SBD) d'image aux rayons X segmentées à des données (RBD) d'image de référence s'effectue à l'aide d'une structure anatomique détectée intéressante dans les données (SBD) d'image aux rayons X segmentées.

3. Procédé suivant la revendication 2, dans lequel la comparaison s'effectue sur la base d'une structure anatomique détectée intéressante dans les données (SBD) d'image aux rayons X segmentées aux données (RBD) d'image de référence à l'aide d'au moins l'un des critères suivants :
- forme de la structure,
- dimension de la structure,
- répartition d'absorption dans la structure,
- existence d'implants dans la structure et type des implants,
- apparition de cas de morts, fractures,
- contexte d'image,
- répartition des degrés de gris dans l'image,
- paramètres d'enregistrement.

4. Procédé suivant l'une des revendications 1 à 3, dans lequel la comparaison s'effectue sur la base d'une structure anatomique détectée intéressante dans les données (SBD) d'image aux rayons X segmentées aux données (RBD) d'image de référence à l'aide de données cliniques du patient.

5. Procédé suivant l'une des revendications précédentes, dans lequel, lors de la détermination de données (RBD) d'image de référence venant immédiatement ensuite, on détermine un ou plusieurs jeux (RBD) de données d'image de référence et on ne rejette pas les données (BD) d'image aux rayons X reçues si au moins l'un des jeux (RBD) de données d'image de référence n'a pas été rejeté.

6. Procédé suivant l'une des revendications précédentes, dans lequel la détermination de données (RBD) d'image de référence venant immédiatement ensuite s'effectue à l'aide d'un procédé de comparaison automatisé.

7. Procédé suivant l'une des revendications précédentes, dans lequel la décision si les données (BD) d'image aux rayons X reçues sont rejetées est prise en fonction du point de savoir si un réglage de paramètres d'une opération d'enregistrement d'image a été modifié.

8. Procédé suivant l'une des revendications précédentes, dans lequel, dans le cas où il a été décidé que les données (BD) d'image aux rayons X reçues sont rejetées, on transmet à l'utilisateur l'une des informations suivantes :
- informations concernant le fondement de la décision,
- instructions (BA) d'utilisateur pour modifier la mise en position du patient et/ou de parties d'un dispositif (30) d'imagerie aux rayons X utilisé pour l'enregistrement des données d'image,
- données statistiques sur des données (BD) d'image rejetées comme avertissement pour l'enregistrement à venir de données (BD) d'image aux rayons X correspondantes.

9. Procédé d'imagerie comportant les stades :
- relevé de données (RD) de projection aux rayons X d'une région à examiner d'un patient à l'aide d'un dispositif (30) d'imagerie aux rayons X,
- production de données (BD) d'image aux rayons X sur la base des données (RD) de projection aux rayons X relevées,
- contrôle des données (BD) d'image aux rayons X en utilisant un procédé suivant l'une des revendications 1 à 8,
- validation des données (BD) d'image aux rayons X pour l'affichage d'image dans le cas où on a déterminé que la qualité des données (BD) d'image aux rayons X est suffisante.

10. Procédé suivant la revendication 9, dans lequel, dans le cas où il a été décidé au stade de contrôle que les données (BD) d'image aux rayons X reçues sont rejetées, on transmet l'une des instructions (AS) suivantes à un dispositif (32) de commande du dispositif (30) d'imagerie aux rayons X :
- instructions de traitement d'image automatisée de l'image rejetée,
- instruction (AS) de correction automatisée de la mise en position du patient et/ou de parties du dispositif (30) d'imagerie aux rayons X.

11. Dispositif (20) de contrôle de données d'image comportant :
- une unité (21) de relevé de données d'image pour la réception de données (BD) d'image aux rayons X d'une région à examiner d'un patient,
- une unité (22) de segmentation pour la segmentation des données (BD) d'image aux rayons X et la détection de structures anatomiques dans les divers segments,
- une unité (23) de comparaison pour la détermination des données (BD) d'image aux rayons X aux données (RBD) d'image de référence venant immédiatement ensuite sur la base d'une comparaison des données (SBD) d'image aux rayons X segmentées à des données (RBD) d'image de référence d'une base (DB) de données de référence, les données (RBD) d'image de référence de la base (DB) de données de référence ayant chacune une information de qualité concernant la qualité d'image des données (RBD) d'image de référence sur le point de savoir si les données (RBD) d'image de référence ont été classées comme pouvant être utilisées pour un diagnostic ultérieur ou comme n'y convenant pas,
- une unité (24) de contrôle pour décider si les données (BD) d'image aux rayons X reçues sont rejetées, sur la base de l'information de qualité des données (RBD) d'image de référence qui ont été déterminées.

12. Dispositif (30) d'imagerie aux rayons X comportant :
- une unité (31) de scan pour relever des données (BD) d'image aux rayons X d'une région à examiner d'un patient,
- un dispositif (32) de commande pour commander l'unité (31) de scan,
- un dispositif (20) de contrôle de données d'image suivant la revendication 11.

13. Produit de programme d'ordinateur ayant un programme d'ordinateur qui peut être chargé directement dans une unité de mémoire d'un dispositif (30) d'imagerie aux rayons X, comprenant des parties de programme pour effectuer tous les stades du procédé suivant l'une des revendications 1 à 10, lorsque le programme d'ordinateur est réalisé dans le dispositif (30) d'imagerie aux rayons X.

14. Support déchiffrable par ordinateur sur lequel sont mémorisées des parties de programme pouvant être réalisées par une unité informatique, afin d'effectuer tous les stades du procédé suivant l'une des revendications 1 à 10, lorsque les parties de programme sont réalisées par l'unité informatique.
